(19) Europäisches Patentamt

European Patent Office

Office européen des brevets

(11) Publication number: **0 337 464 B1**

# EUROPEAN PATENT SPECIFICATION

(45) Date of publication of patent specification: **15.06.94**   (51) Int. Cl.⁵: **A61K 7/38**

(21) Application number: **89106640.9**

(22) Date of filing: **13.04.89**

The file contains technical information submitted
after the application was filed and not included in
this specification

(54) **Antiperspirant and method of making same.**

(30) Priority: **14.04.88 US 181564**

(43) Date of publication of application:
**18.10.89 Bulletin 89/42**

(45) Publication of the grant of the patent:
**15.06.94 Bulletin 94/24**

(84) Designated Contracting States:
**AT BE CH DE ES FR GB GR IT LI NL SE**

(56) References cited:
**EP-A- 0 291 960**
**DE-A- 2 549 464**
**GB-A- 1 568 831**
**GB-A- 2 048 229**

(73) Proprietor: **The Gillette Company**
**Prudential Tower Building**
**Boston, Massachusetts 02190(US)**

(72) Inventor: **Curtin, Maria A.**
**320 Foundry Street**
**South Easton 02375 Massachusetts(US)**
Inventor: **Phipps, Alan M.**
**35 Crest Road**
**Framingham 01701 Massachusetts(US)**

(74) Representative: **Baillie, Iain Cameron et al**
**c/o Ladas & Parry**
**Altheimer Eck 2**
**D-80331 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

This invention relates to a method of providing improved antiperspirant effectiveness and stability for basic aluminum chloride compositions (also known as aluminum chlorhydroxide compositions) and to such improved compositions. It has hitherto been proposed to increase the antiperspirant effectiveness of aluminum chlorhydroxide by aging under specified conditions an aqueous solution containing the aluminum chlorhydroxide, as described in Gosling et al. U.S. Patent 4,359,456 and in British Patent Application No. 2048229A until the solution exhibits specified characteristics. In the British application it is pointed out that the increased activity is the result of a group of complexes called $Al^{c'}$ characterized by having a diffusion constant in gel-permeation chromatography which is within the range generally found for the $Al^b$ group of complexes but which displays a complexing rate in the ferron test which is in the range of the $Al^c$ complexes and by having molecules which are less than 100 A in size in aqueous solution. The $Al^{c'}$ complexes thus prepared are stable in aqueous solution at concentrations in the range of 10% to 30%.

It has also been proposed to increase the antiperspirant effectiveness of aluminum chlorhydroxide compositions by including in them zirconyl hydroxy chloride, and further by subjecting aqueous solutions of such compositions to heating under certain conditions, then rapidly drying the solution to solid form. The product when dissolved in water loses its improved efficacy rapidly, within a matter of hours.

It has now been found that a large increase in antiperspirant effectiveness of basic aluminum chloride (or aluminum chlorhydroxide) is provided by mixing it with monisilicic acid in aqueous solution , the amount of the chloride being 5-20% by weight and the amount of monosilicic acid being 0.03 to 8% by weight, preferably 0.3 to 3%, based upon the weight of the total mixed solution. The molecular distribution characteristics of the basic aluminum chloride immediately begin to change as determined by size exclusion (SE) chromatography, and at the same time the antiperspirant effectiveness begins to improve; the improvement continues at a gradually decreasing rate at room temperature for a period of a week or more, after which the composition retains its improved efficacy indefinitely, whether the composition is in the form of an aqueous solution or whether it is dried to a solid by removing the solvent. In dry solid form the composition contains basic aluminum chloride and silicic acid in proportions as low as a few parts per million of the latter, based on the total. Excess silicic acid in any amount added after the desired increase in antiperspirant effectiveness has been achieved has no effect.

In the drawings,

Fig. 1 shows a generalized chromatogram of a representative product of the present invention;

Fig. 2 shows a chromatogram of the product of Example 1 of the present invention;

Fig. 3 shows a chromatogram of the basic aluminum chloride starting material used in Example 1;

Fig. 4 shows a chromatogram of the product of Example 2 of the present invention;

Fig. 5 shows a chromatogram of the product of Example 3 of the present invention; and

Fig. 6 shows a chromatogram of the basic aluminum chloride starting material used in Examples 2, 3 and 4.

The monosilicic acid in solution should be freshly prepared; by this is meant an aqueous solution containing the acid in dissolved form, substantially free from precipitate and from gel. The precise amount of monosilicic acid required to convert conventional basic aluminum chloride to the new form having greatly enhanced antiperspirant effectiveness varies depending not only upon the concentration of the chloride but upon the procedure used to prepare the monosilicic acid solution and upon the length of time elapsing between its preparation and its use. For optimum results, the solution of monosilicic acid should be used as soon as possible after its preparation. Depending upon temperature of storage as well as upon pH and concentration, the monosilicic acid solution tends to lose its effectiveness after storage for a few days or weeks, presumably because of progressive polymerization of the monosilicic acid. Water-insoluble or precipitated silica, e.g. colloidal silica, or silica gel, is ineffective for the purpose of this invention, as is an alkaline solution of a silicate such as sodium silicate.

It has been reported in the prior art that freshly prepared aqueous solutions of silicic acid exist in "true solution". See Otterstedt et al., J. Colloid and Interface, Sci., Vol. 115(1), 95-103 (1987) and Iler et al., J.Phys.Chem., Vol. 57, 604 (1953). These solutions are initially composed of monomeric silicic acid units. The species encountered in these solutions appear to be labile and changes in concentration or pH are followed by rapid changes in species distribution and polymerization. The silicic acid will eventually precipitate as silica until the soluble species are reduced to about $10^{-3}M$ (the solubility constant for amorphous silica).

The rate of silica formation and precipitation depends upon initial concentration of the monosilicic acid in solution, pH, temperature, the presence of cations other than hydrogen, and the presence of miscible solvent other than water, such as lower alkanols. Increases in any of the foregoing except the last tend to increase the instability of the solution and accelerate precipitation or gel formation. The presence of basic aluminum chloride dissolved in the solution of monosilicic acid increases the pH to about 3.5 to 4.

While applicants do not wish to be bound to a particular theory of interaction between the basic aluminum chloride and monosilicic acid, it is believed that competition between the monosilicic acid and the basic aluminum chloride for hydroxide ions in aqueous solution at a pH from 2 to 4, when the solution contains a very low concentration of free hydroxide ions, causes redistribution in the basic aluminum chloride to provide the molecular form which displays increased antiperspirant effectiveness.

The monosilicic acid solution can be prepared by various conventional procedures, such as the one described in Alexander, J.Am.Chem.Soc., Vol. 75, pp. 2887-8 (1953). In one preferred embodiment of the invention it is prepared by treating an aqueous solution of soluble silicate salt such as sodium or potassium silicate with a water-insoluble cation exchange resin in its hydrogen form as described, for example, in U.S. Patent No. 2,588,389. In another preferred embodiment it is prepared by hydrolysis of tetraethyl orthosilicate. Mixing or stirring of tetraethyl orthosilicate with water at room temperature suffices to produce hydrolysis, but heating accelerates the hydrolysis as does the addition of an acid, e.g. a mineral acid such as hydrochloric.

The presence of zirconyl hydroxy chloride along with basic aluminum chloride, either during or after treatment of the latter in accordance with the present invention is not deleterious. The basic aluminum chloride may be added in dry powder form to the freshly prepared monosilicic acid aqueous solution with stirring, or if desired the basic aluminum chloride in aqueous solution may be mixed with the acid solution.

The time required to achieve optimum conversion of basic aluminum chloride to the desired product after mixing with an aqueous monosilicic acid solution may vary from several hours to several weeks, depending upon the nature of the preparation of the monosilicic acid, the extent and conditions of its storage before use; and the relative concentrations of monosilicic acid and of the basic aluminum chloride.

Once the desired conversion has been achieved, the water and other volatile components such as alcohol, if present, may be evaporated if desired. The length of time the solution is allowed to stand before drying, as well as the speed of drying are not critical since the product continues to maintain indefinitely its superior antiperspirant effectiveness, once attained, whether it is in solution or in the form of dry particles, and at moderately elevated temperatures as well as at lower temperatures, even below 0 °C. If colloidal or precipitated silica has formed in the mixed solution it may be removed, by filtration for example. Although some decrease in antiperspirant effectiveness may result, whether or not the composition is subsequently dried to solid form. Even after filtration residual silicic acid remains in the composition, either in the form of monosilicic acid or as its polymerization products although its amount is greatly reduced from the proportion present in the initial mixture of basic aluminum chloride and monosilicic acid and may even be as low as of the order of parts per million of the basic aluminum chloride. The addition of colloidal silica or silica gel to the composition also has no effect upon the antiperspirant effectiveness of the composition.

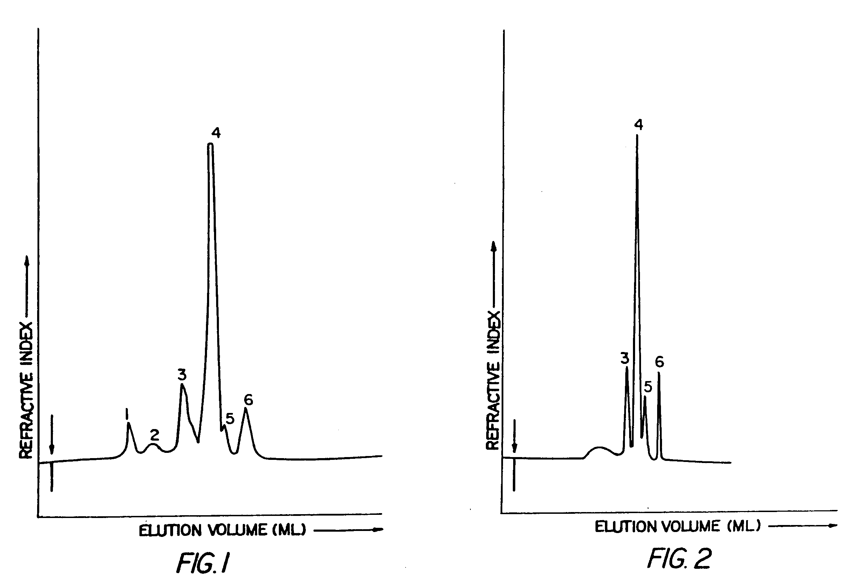

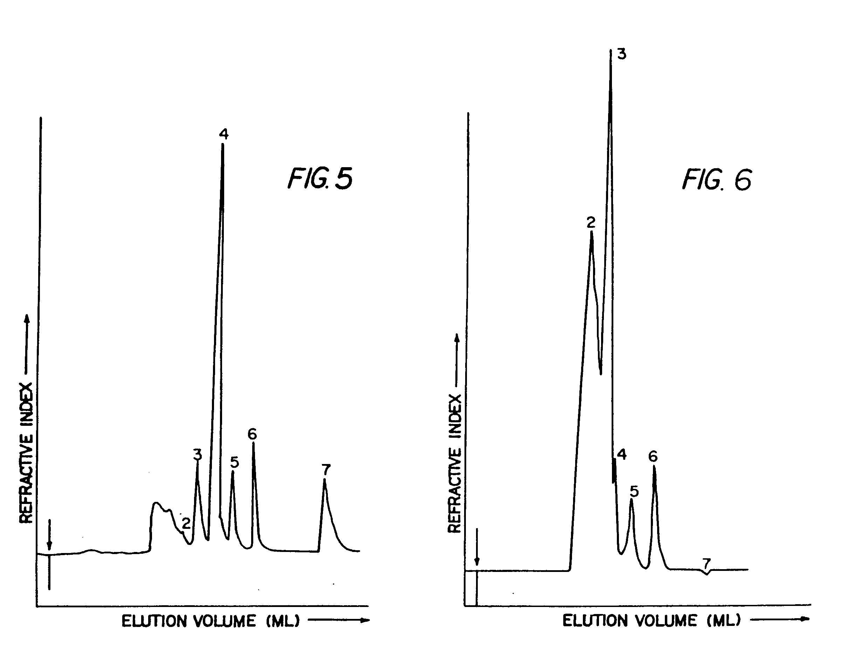

The new product having superior antiperspirant effectiveness is characterized and distinguished from other forms of basic aluminum chloride of relatively low antiperspirant effectiveness by its size exclusion chromatogram, its silicic acid content, and by the fact that its antiperspirant effectiveness does not decrease in contact with water. The chromatogram is obtained by subjecting to size exclusion chromatography a specimen of a water solution containing approximately 10% by weight of the basic aluminum chloride and silicic acid. The solution is filtered through a membrane filter having 0.45μm diameter pores, then applied to a size exclusion (SE) HPLC column and eluted, and the refractive index of successive fractions of the eluent as it leaves the column is measured and plotted against the cumulative total volume of eluent to provide the chromatogram. Each peak in the chromatogram indicates the presence of a different molecular size component of the solution in the fraction of eluent being measured. A representative chromatogram of the product of the present invention on a column of Sephadex G-50 (as defined below) is illustrated in Fig. 1, in which the fraction exhibiting the first peak(1) contains colloidal silica. Most products will show only a very small peak 1 because most of the free silica precipitates and is removed by the filtration step prior to chromatography. In some cases this peak can be resolved into two or more separate peaks. The fractions of peaks 2-5 all contain aluminum compounds. Peak 2 fraction contains unchanged or residual basic aluminum chloride. Peak 3 and 4 fractions together contain at least 80% of the total aluminum compounds present in the specimen and display improved antiperspirant effectiveness as compared to the starting material of peak 2; peak 4 is at least twice the height of peak 3, preferably four times as high or higher. Peak 5 fraction contains low molecular weight aluminum species sometimes present in commercially available basic aluminum chloride; the height of peak 5 increases with decrease in the pH of the eluent, but it remains a minor component in every case. Peak 6 fraction contains little or no aluminum. Some of the peaks, particularly those displaying shoulders, may be separated into two or more peaks by increasing the resolution of the chromatograph. When other ingredients are present, such as the ethyl alcohol by-product of hydrolysis of tetraethyl orthosilicate, they may form additional peaks, as shown in the chromatogram of Fig. 5, depending upon the column used.

The location of the fractions of peaks 3 and 4 of any specimen can be identified in terms of the distribution constant or value, defined as:

$$K = \frac{V - V_e}{V_t - V_e}$$

where:

$V$ = Volume of eluent which has passed through the column at the point at which the particular peak appears on the chromatogram.

$V_e$ = Volume of eluent which has passed at the point at which the "excluded peak" appears on the chromatogram. The "excluded peak", which is peak 1 of Fig. 1, contains all of the material of the specimen being examined which is larger than the size exclusion limit of the packing material of the column.

$V_t$ = Volume which has proceeded through the column at the point at which the "totally included" peak appears; the totally included peak (peak 6 of Fig. 1) contains the material of the specimen being examined which is of such a molecular size that it is totally included in the pore volume of the column.

These K values will vary with the type of column material and eluent used. For example, the following column materials can be used:

A. porous spherical silica particles approximately 5 $\mu$m in diameter having a pore diameter of 10 nm. (for example, Nucleosil 100-5 or 100-7 from Machery Nagel);

B. silica with glycerylpropyl bonded phase particles approximately 5 $\mu$m in diameter having a pore diameter of 6 nm and having a molecular weight range of 20,000 (excluded) to 300 (included) for globular proteins (for example, Synchropak GPC-60 from Synchron. Inc.);

C. a cross-linked dextran having a molecular weight range of 30.000 (excluded) to 1000 (included) for globular proteins (for example, Sephadex G50).

The eluents for the three columns are as follows:

Column A:    0.01 M aqueous nitric acid

Column B:    0.1 M aqueous potassium chloride adjusted to pH 4.5 with hydrochloric acid

Column C:    0.1 M aqueous potassium chloride adjusted to pH 3 with hydrochloric acid

The K values for the two peaks indicating the fractions which are characteristic of the new products are as follows for the three columns and eluents:

A. 0.30-0.40 and 0.49-0.53

B. 0.27-0.29 and 0.31-0.34

C. 0.47-0.53 and 0.68-0.72

In each case, the height of the peak having the larger K value is at least twice the height of the other in all of the products of the present invention. Column dimensions, specimen size, and eluent flow rate depend on the system used. In the case of column A, length was 50 cm, diameter 4.6 mm stainless steel, specimen size was 2-4 $\mu$L, and eluent flow rate was 0.5-0.75 mL/min. For column B, length was 30 cm, diameter 4.6 mm. stainless steel, specimen size was 2 $\mu$L, and eluent flow rate was 0.4 ml/min. For column C, length was 96 cm, diameter 9 mm glass, specimen size was 100 $\mu$L, and eluent flow rate was 0.2 ml/min.

The stability of the product of the present invention is such that its effectiveness as an antiperspirant does not decrease when it is stored either in dry solid form or as an aqueous solution; an improvement in antiperspirant effectiveness of the aqueous solution does occur during a period of the first week or more at room temperature after initial mixing of basic aluminum chloride with monosilicic acid solution, however, but at a decreasing rate, so that once it has reached maximum effectiveness it remains unchanged during further storage in either dry form or as an aqueous solution for several months or more at room temperature. This stability is reflected also in the chromatogram of the product; although the ratio of the height of peak 4 to that of peak 3 in Fig. 1 increases when the product remains in aqueous solution at room temperature, the rate of increase slows with the lapse of time after preparation of the mixed solution. When the product is in dry solid form it remains unchanged indefinitely at room temperature; water solutions made from the dry solid display the same chromatogram and same antiperspirant effectiveness regardless of the length of time the solid product has been stored. Consequently, the products of the present invention display high antiperspirant efficacy without decrease in contact with water; they also display, in solution in

4

water at approximately 10% by weight, a chromatogram in which the ratio of the height of peak 4 to that of peak 3 does not decrease.

The high stability of the product of the present invention both in dry solid form and, within a few hours after the initial mixing step, in the form of an aqueous solution, makes it possible to incorporate it as the principal or sole active antiperspirant agent in a wide variety of conventional formulations including powders, creams, sticks, solutions, and dispersions or emulsions, including aerosol compositions. The formulations may be dispensed from any conventional containers such as roll-on applicators, aerosol cans, and solid stick containers. Once the composition has been dried, it is desirable to avoid using more than about 1.5 - 2% ethyl alcohol in formulations containing it because the alcohol tends to cause a decrease in antiperspirant effectiveness as shown by a change in the chromatogram.

Typical antiperspirant formulations in which the material of the present invention may be practically employed in anhydrous form are as follows:

I. Non-aqueous Roll-On

|  | Weight % |
|---|---|
| Finely divided solid of present invention | 20.0 |
| Quaternium-18 Hectorite | 2.7 |
| Anhydrous Alcohol SDA-40 | 1.6 |
| H20 | 0.2 |
| Cyclomethicone, a silicone oil | 75.5 |
| Perfume | q.s |

II. Stick Antiperspirant

|  | Weight % |
|---|---|
| Finely divided solid of present invention | 23.0 |
| Ozokerite Wax | 22.4 |
| Myristyl Alcohol | 17.2 |
| Cyclomethicone | 17.9 |
| PPG-15 Stearyl ether | 11.5 |
| Steareth-15 | 2.3 |
| Bentone Gel IPM | 5.7 |
| Perfume | q.s |

III. Aerosol Antiperspirant

|  | Weight % |
|---|---|
| Finely divided solid of present invention | 6.8 |
| Propylene Carbonate | 0.3 |
| Quaternium-18 Hectorite | 1.0 |
| Cyclomethicone | 12.4 |
| Isopropyl myristate | 2.0 |
| Perfume | 0.6 |
| Propellant-A31 | 76.9 |

In addition, the anhydrous solid of the present invention can be replaced by an aqueous solution of the material, as, for example, in the following case:

IV. Aqueous Roll-On

|  | Weight % |
|---|---|
| Material of the present invention as a 15% aqueous solution | 73.0 |
| Cyclomethicone | 20.0 |
| Steareth-2 | 2.2 |
| Steareth-21 | 1.0 |
| PPG-15 Stearyl ether | 1.8 |
| Aluminum Starch Octenyl succinate | 2.0 |

The following specific examples are intended to illustrate more fully the nature of the present invention.

Example 1

A 200 g portion of ion-exchange resin (Dowex HCR-S) was washed with 1 M hydrochloric cid, then with distilled water and air-dried. The resulting cationic exchange resin was slurried in 300 ml of 0.05 M hydrochloric acid and cooled to below 10°C in a water-ice bath. There was dissolved in the slurry 12•g of sodium metasilicate nonahydrate ($Na_2SiO_3.9 H_2O$) in the form of finely ground powder. The exchange resin was then removed by filtration leaving a clear solution of monosilicic acid approximately 0.14M in concentration at a pH of 2.2. To a 270 g portion of the solution was added a 30 g portion of basic aluminum chloride in dry powder form prepared by heating a 10% solution of basic aluminum chloride in water at 80°C for 16 hours and spray drying, as described in UK patent Application 2,048,229A published December 10, 1980. The resulting mixed solution containing 10% by weight of basic aluminum chloride was allowed to stand at room temperature for 48 hours.

An aliquot of the solution was filtered through a membrane having 0.45 μm diameter pores and was then subjected to SE HPLC on a column containing Nucleosil 100-7 using as eluent 0.01 M nitric acid and measuring refractive index. The resulting chromatogram is shown in Fig. 2 of the drawing, the second principal peak (4) having a height approximately 3.3 times that of the first (3).

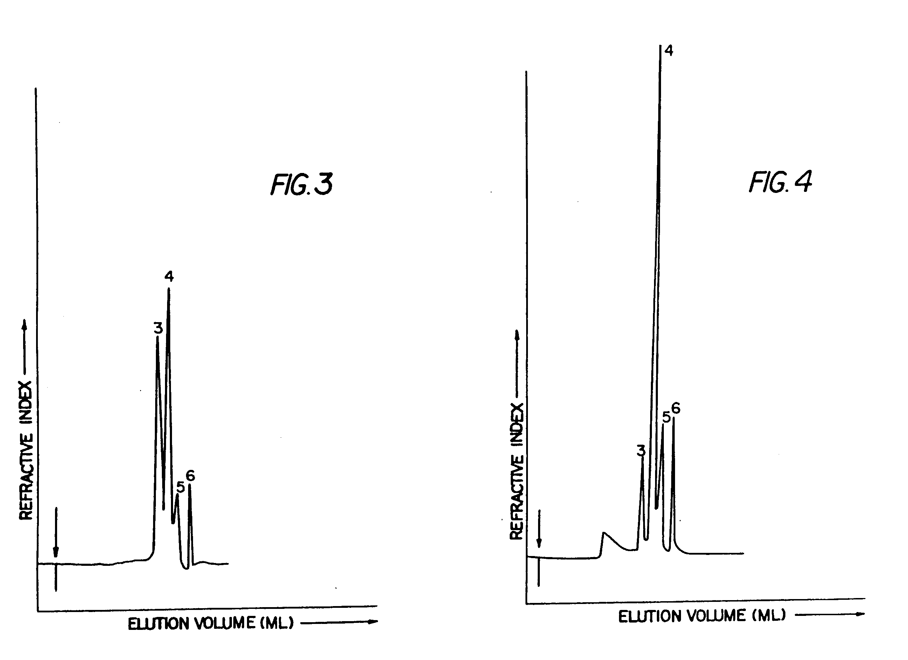

As a control another 30 g portion of the same basic aluminum chloride dry powder starting material was dissolved in 270 g distilled water, allowed to stand 48 hours at room temperature, and subjected to SE HPLC at intervals under the same conditions as above. The ratio of the height of peak 4 to that of peak 3 decreased from the initial value. The chromatogram of the resulting solution is shown in Fig. 3, in which the ratio of the height of the second peak to that of the first is approximately 1.2:1.

Separate aliquots of the two solutions were tested for sweat reduction effectiveness on a panel of test subjects under standard conditions. The solution of the product of the invention was found to be significantly more effective than the control solution.

Example 2

A slurry of cation-exchange resin in 0.05 M hydrochloric acid was prepared as described in Example 1 and cooled to below 10°C. To the slurry was added slowly with stirring 30.6 g of finely ground sodium metasilicate nonahydrate and allowed to dissolve; the slurry was then filtered to remove the resin and provide a solution of monosilicic acid approximately 0.27 M in concentration.

Commercially available basic aluminum chloride was dissolved in water to provide a 50% by weight solution. An aliquot of this solution, after dilution to approximately 10% by weight, exhibited the chromatogram shown in Fig. 6 in which the ratio of the height of peak 4 to that of peak 3 is approximately 0.23:1.

Into 64 g of the monosilicic acid solution there was mixed by stirring 16 g of the 50% basic aluminum chloride solution and the mixture was allowed to warm to room temperature. After many days a portion was subjected to SE HPLC as described in Example 1 and was found to exhibit the chromatogram shown in Fig. 4 in which the height of the second principal peak (4) was approximately 5 times that of the first (3).

The mixture, in the form of an aqueous solution, exhibited significantly more antiperspirant effectiveness than did an aqueous solution containing the same concentration of commercially available basic aluminum chloride alone. It was essentially equal in antiperspirant effectiveness to an aqueous solution containing dissolved commercially available basic aluminum chloride together with zirconyl hydroxy chloride (atomic ratio of Al:Zr approximately 3.6 to 1) and glycine as an antigelling agent.

Example 3

There were dissolved in 100 g of water 100 g of the same basic aluminum chloride used as starting material in Example 2. This solution was mixed with 750 ml of distilled water; 42.8 ml of tetraethylorthosilicate [TEOS] (Reagent grade, Fisher Scientific Co.) were added to the mixed solution, and the mixture was stirred for four weeks at room temperature. The solution became viscous and opaque after a couple of weeks due to the formation of a silica sol.

An HPLC chromatogram of the final product after filtration through a 0.45 $\mu$m pore diameter filter is shown in Figure 5. The peak height ratio of peak 4 to peak 3 (the peaks of interest) is 4.4. In this case the peak preceding peak 2 is resolved into two peaks, both containing colloidal silica of such small particle size that it was not removed by the filtration step. Peaks 5 and 6 are due to small aluminum species and chloride and do not change during the reaction. The last peak observed, peak 7, contains ethanol, a by-product of TEOS hydrolysis.

The above solution exhibited significantly more antiperspirant effectiveness than did an aqueous solution containing the same concentration of basic aluminum chloride prepared as described in U.K. Patent Application 2,048,229A published December 10, 1980.

Example 4

A water solution containing 50% by weight of commercially available basic aluminum chloride was prepared, and a 200 g portion was mixed with 780 grams of distilled water and 10 ml of 6 N HC1. The solution was heated for two and a half hours at 80°C, then 22 ml of tetraethylorthosilicate [TEOS] were added to the solution with continuous stirring and heating at 80°C for another hour.

The resulting solution was slightly opaque; this solution was then spray dried to obain a white powder. An SE HPLC chromatogram of the powder redissolved in water at a concentration of 10% by weight contained a peak 4 to peak 3 ratio of 2.2; and a chromatogram similar to that shown in Fig. 2. The ethanol peak was absent since the ethanol was evaporated during the spray drying process.

The powder obtained by this method when formulated in the non-aqueous roll-on formulation base described above exhibited significantly more antiperspirant effectiveness than did the same formulation base containing basic aluminum chloride powder prepared as described in U.K. Patent Application 2,048,229A.

**Claims**
**Claims for the following Contracting States : AT, BE, CH, DE, FR, GB, GR, IT, LI, NL, SE**

1.  A method of increasing the antiperspirant efficacy of basic aluminum chloride, which comprises mixing said chloride with monosilicic acid in aqueous solution, the amount of said chloride being from 5% to 20% by weight and the amount of said acid being from 0.03% to 8% by weight based on the weight of said mixed solution.

2.  The method as claimed in claim 1, in which the mixing is carried out at a temperature from 5 to 100°C., preferably at room temperature to 80°C.

3.  The method as claimed in claim 1 or 2, in which the pH of said mixed solution is from 2 to 4.

4.  The method as claimed in any one of claims 1 to 3, in which the amount of said acid is from 0.3 to 3%.

5.  The method as claimed in any one of claims 1 to 4, wherein said monosilicic acid is prepared in situ by hydrolysis of tetraethylorthosilicate.

6.  The method as claimed in any one of claims 1 to 5, additionally comprising adding zirconyl hydroxy chloride.

7.  The method as claimed in any one of claims 1 to 6, comprising the additional step of drying said mixed solution to provide the solute in solid form.

8.  A composition comprising basic aluminum chloride and silicic acid, said composition displaying high antiperspirant efficacy without decrease in contact with water, wherein a chromatogram of an approximately 10% aqueous solution thereof made by size exclusion chromatography displays two successive peaks corresponding to peaks 3 and 4 as shown in Fig. 2, said peaks containing at least 80% of the total aluminum in said composition, the ratio of the height of peak 4 to that of peak 3 being at least 2:1, said size exclusion chromatography being performed by passing a 2-4 µL sample through a 50 cm long stainless steel column of 4.6mm diameter filled with porous spherical silica particles of approximately 5µm diameter having a pore diameter of 10nm using 0.01M aqueous nitric acid as eluent at a flow rate of 0.5-0.75 mL/min.

9. A composition as claimed in claim 9 in the form of an aqueous solution.

**Claims for the following Contracting State : ES**

1. A method of increasing the antiperspirant efficacy of basic aluminum chloride, which comprises mixing said chloride with monosilicic acid in aqueous solution, the amount of said chloride being from 5% to 20% by weight and the amount of said acid being from 0.03% to 8% by weight based on the weight of said mixed solution.

2. The method as claimed in claim 1, in which the mixing is carried out at a temperature from 5 to 100°C., preferably at room temperature to 80°C.

3. The method as claimed in claim 1 or 2, in which the pH of said mixed solution is from 2 to 4.

4. The method as claimed in any one of claims 1 to 3, in which the amount of said acid is from 0.3 to 3%.

5. The method as claimed in any one of claims 1 to 4, wherein said monosilicic acid is prepared in situ by hydrolysis of tetraethylorthosilicate.

6. The method as claimed in any one of claims 1 to 5, additionally comprising adding zirconyl hydroxy chloride.

7. The method as claimed in any one of claims 1 to 6, comprising the additional step of drying said mixed solution to provide the salute in solid form.

8. A method according to any one of claims 1 to 6, which comprises obtaining the salute in the form of an aqueous solution.

9. A method according to claim 8, wherein a chromatogram of an approximately 10% aqueous solution made by size exclusion chromatography displays two successive peaks corresponding to peaks 3 and 4 as shown in Fig. 2, said peaks containing at least 80% of the total aluminum in said composition, the ratio of the height of peak 4 to that of peak 3 being at least 2:1, said size exclusion chromatography being performed by passing a 2-4 µL sample through a 50 cm long stainless steel column of 4.6mm diameter filled with porous spherical silica particles of approximately 5µm diameter having a pore diameter of 10nm using 0.01M aqueous nitric acid as eluent at a flow rate of 0.5-0.75 mL/min.

**Patentansprüche**
**Patentansprüche für folgende Vertragsstaaten : AT, BE, CH, DE, FR, GB, GR, IT, LI, NL, SE**

1. Verfahren zum Erhöhen der antihydrotischen Wirksamkeit von basischem Aluminiumchlorid, welches umfaßt: Mischen des Chlorids mit Orthokieselsäure in wäßriger Lösung, wobei die Menge des Chlorids von 5 % bis 20 Gew.% und die Menge der Säure von 0,03 % bis 8 Gew.% bezogen auf das Gewicht der gemischten Lösung beträgt.

2. Verfahren nach Anspruch 1, bei welchem das Mischen bei einer Temperatur von 5 °C ... 100 °C, vorzugsweise bei Raumtemperatur bis 80 °C, ausgeführt wird.

3. Verfahren nach Anspruch 1 oder 2, bei welchem der pH-Wert der gemischten Lösung 2 bis 4 beträgt.

4. Verfahren nach einem der Ansprüche 1 bis 3, bei welchem die Menge der Säure 0,3 % bis 3 Gew.% beträgt.

5. Verfahren nach einem der Ansprüche 1 bis 4, bei welchem die Orthokieselsäure durch Hydrolyse von Tetraethylorthosilicat in situ hergestellt wird.

6. Verfahren nach einem der Ansprüche 1 bis 5, zusätzlich umfassend das Zusetzen von Zirconylhydroxychlorid.

7. Verfahren nach einem der Ansprüche 1 bis 6, umfassend den zusätzlichen Schritt des Trocknens in der gemischten Lösung, um den gelösten Stoff in fester Form zu schaffen.

8. Zusammensetzung, umfassend basisches Aluminiumchlorid und Kieselsäure, welche Zusammensetzung hohe antihydrotische Wirksamkeit ohne Verringerung bei Kontakt mit Wasser zeigt, bei welcher ein durch Ausschlußchromatographie erhaltenes Chromatogramm einer näherungsweise 10%igen wäßrigen Lösung von ihr zwei aufeinanderfolgende Peaks entsprechend den Peaks 3 und 4 in Fig. 2 zeigt, welche Peaks mindestens 80 % des gesamten Aluminiums in der Zusammensetzung enthalten, und das Verhältnis der Höhe von Peak 4 zu der von Peak 3 mindestens 2:1 beträgt, wobei die Ausschlußchromatographie durchgeführt wird, indem eine 2 bis 4 ml-Probe durchläuft durch eine 50 cm lange Säule aus rostfreiem Stahl mit einem Durchmesser von 4,6 mm, gefüllt mit porösen, kugelförmigen Siliciumdioxid-Teilchen mit einem Durchmesser von näherungsweise 5 Mikrometer und einem Porendurchmesser von 10 nm, und unter Verwendung von 0,01 m wäßriger Salpetersäure als Eluant bei einer Durchflußrate von 0,5 ... 0,75 ml/min.

9. Zusammensetzung nach Anspruch 9 in Form einer wäßrigen Lösung.

**Patentansprüche für folgenden Vertragsstaat : ES**

1. Verfahren zum Erhöhen der antihydrotischen Wirksamkeit von basischem Aluminiumchlorid, welches umfaßt: Mischen des Chlorids mit Orthokieselsäure in wäßriger Lösung, wobei die Menge des Chlorids von 5 % bis 20 Gew.% und die Menge der Säure von 0,03 % bis 8 Gew.% bezogen auf das Gewicht der gemischten Lösung beträgt.

2. Verfahren nach Anspruch 1, bei welchem das Mischen bei einer Temperatur von 5 °C ... 100 °C, vorzugsweise bei Raumtemperatur bis 80 °C, ausgeführt wird.

3. Verfahren nach Anspruch 1 oder 2, bei welchem der pH-Wert der gemischten Lösung 2 bis 4 beträgt.

4. Verfahren nach einem der Ansprüche 1 bis 3, bei welchem die Menge der Säure 0,3 % bis 3 Gew.% beträgt.

5. Verfahren nach einem der Ansprüche 1 bis 4, bei welchem die Orthokieselsäure durch Hydrolyse von Tetraethylorthosilicat in situ hergestellt wird.

6. Verfahren nach einem der Ansprüche 1 bis 5, zusätzlich umfassend das Zusetzen von Zirconylhydroxychlorid.

7. Verfahren nach einem der Ansprüche 1 bis 6, umfassend den zusätzlichen Schritt des Trocknens in der gemischten Lösung, um den gelösten Stoff in fester Form zu schaffen.

8. Verfahrennach Anspruch 1 bis 6, elches umfaßt, den gelösten Stoff in Form einer wässrigen Lösung zu erhalten.

9. Verfahren nach Anspruch 8, bei welchem ein durch Ausschlußchromatographie erhaltenes Chromatogramm einer näherungsweise 10%igen wäßrigen Lösung von zwei aufeinanderfolgende Peaks entsprechend den Peaks 3 und 4 in Fig. 2 zeigt, welche Peaks mindestens 80 % des gesamten Aluminiums in der Zusammensetzung enthalten, und das Verhältnis der Höhe von Peak 4 zu der von Peak 3 mindestens 2:1 beträgt, wobei die Ausschlußchromatographie durchgeführt wird, indem eine 2 bis 4 ml-Probe durchläuft durch eine 50 cm lange Säule aus rostfreiem Stahl mit einem Durchmesser von 4,6 mm, gefüllt mit porösen, kugelförmigen Siliciumdioxid-Teilchen mit einem Durchmesser von näherungsweise 5 Mikrometer und einem Porendurchmesser von 10 nm, und unter Verwendung von 0,01 m wäßriger Salpetersäure als Eluant bei einer Durchflußrate von 0,5 ... 0,75 ml/min.

**Revendications**

**Revendications pour les Etats contractants suivants : AT, BE, CH, DE, FR, GB, GR, IT, LI, NL, SE**

1. Méthode pour augmenter l'efficacité antiperspirante du chlorure d'aluminium basique qui comprend le mélange dudit chlorure avec l'acide monosilicique en solution aqueuse, la quantité dudit chlorure étant de 5 % à 20% en masse et la quantité dudit acide étant de 0,03 % à 8 % en masse, quantités exprimées par rapport à la masse de ladite solution mélangée.

2. Méthode selon la revendication 1, dans laquelle le mélange est réalisé à une température allant de 5 à 100°C, de préférence de la température ambiante à 80°C.

3. Méthode selon la revendication 1 ou 2, dans laquelle le pH de ladite solution mélangée va de 2 à 4.

4. Méthode selon une quelconque des revendications 1 à 3, dans laquelle la quantité dudit acide va de 0,3 à 3 %.

5. Méthode selon une quelconque des revendications 1 à 4, dans laquelle ledit acide monosilicique est préparé in situ par hydrolyse de l'orthosilicate de tétraéthyle.

6. Méthode selon une quelconque des revendications 1 à 5, comprenant additionnellement l'addition de l'hydroxychlorure de zirconyle.

7. Méthode selon une quelconque des revendications 1 à 6, comprenant l'étape additionnelle de séchage de ladite solution mélangée pour donner le soluté sous forme solide.

8. Composition comprenant du chlorure d'aluminium basique et de l'acide silicique, ladite composition présentant une efficacité antiperspirante élevée, ne diminuant pas au contact de l'eau, un chromatogramme d'une solution aqueuse approximativement à 10 % de cette composition réalisé par chromatographie d'exclusion de tailles présentant deux pics successifs correspondants aux pics 3 et 4 tels que figurant dans la figure 2, lesdits pics contenant au moins 80 % de la totalité de l'aluminium de ladite composition, le rapport de la hauteur du pic 4 à celle du pic 3 étant au moins de 2:1, ladite chromatographie d'exclusion de tailles étant réalisée en faisant passer un échantillon de 2-4 $\mu$l au travers d'une colonne en acier inoxydable de 50 cm de long et de 4,6 mm de diamètre, garnie de particules sphériques poreuses de silice ayant approximativement 5 $\mu$m de diamètre et ayant des pores de 10 nm de diamètre, en utilisant comine éluant de l'acide nitrique aqueux 0,01 M à un débit de 0,5-0,75 ml/mn.

9. Composition selon la revendication 9 sous la forme d'une solution aqueuse.

**Revendications pour l'Etat contractant suivant : ES**

1. Méthode pour augmenter l'efficacité antiperspirante du chlorure d'aluminium basique qui comprend le mélange dudit chlorure avec l'acide monosilicique en solution aqueuse, la quantité dudit chlorure étant de 5 % à 20 % en masse et la quantité dudit acide étant de 0,03 % à 8 % en masse, quantités exprimées par rapport à la masse de ladite solution mélangée.

2. Méthode selon la revendication 1, dans laquelle le mélange est réalisé à une température allant de 5 à 100°C, de préférence de la température ambiante à 80°C.

3. Méthode selon la revendication 1 ou 2, dans laquelle le pH de ladite solution mélangée va de 2 à 4.

4. Méthode selon une quelconque des revendications 1 à 3, dans laquelle la quantité dudit acide va de 0,3 à 3 %.

**EP 0 337 464 B1**

**5.** Méthode selon une quelconque des revendications 1 à 4, dans laquelle ledit acide monosilicique est préparé in situ par hydrolyse de l'orthosilicate de tétraéthyle.

**6.** Méthode selon une quelconque des revendications 1 à 5, comprenant additionnellement l'addition de l'hydroxychlorure de zirconyle.

**7.** Méthode selon une quelconque des revendications 1 à 6, comprenant l'étape additionnelle de séchage de ladite solution mélangée pour donner le soluté sous forme solide.

**8.** Méthode selon une quelconque des revendications 1 à 6, qui comprend l'obtention du soluté sous la forme d'une solution aqueuse.

**9.** Méthode selon la revendication 8, dans laquelle un chromatogramme d'une solution aqueuse approximativement à 10 % réalisé par chromatographie d'exclusion de tailles présente deux pics successifs correspondants aux pics 3 et 4 tels que figurant dans la figure 2, lesdits pics contenant au moins 80 % de la totalité de l'aluminium de ladite composition, le rapport de la hauteur du pic 4 à celle du pic 3 étant au moins de 2:1, ladite chromatographie d'exclusion de tailles étant réalisée en faisant passer un échantillon de 2-4 $\mu$l au travers d'une colonne en acier inoxydable de 50 cm de long et de 4,6 mm de diamètre, garnie de particules sphériques poreuses de silice ayant approximativement 5 $\mu$m de diamètre et ayant des pores de 10 nm de diamètre, en utilisant comme éluant de l'acide nitrique aqueux 0,01 M à un débit de 0,5-0,75 ml/mn.

FIG. 1

FIG. 2

FIG. 4

FIG. 3

FIG. 5

FIG. 6

REFRACTIVE INDEX

ELUTION VOLUME (ML)

EP 0 337 464 B1